# EUROPÄISCHE PATENTSCHRIFT

(11) **EP 3 544 547 B1**
(45) Veröffentlichungstag und Bekanntmachung des Hinweises auf die Patenterteilung: **29.07.2020**
(21) Anmeldenummer: 18707334.1
(22) Anmeldetag: 22.02.2018
(51) Int. Cl.: A61F 2/20

(54) **EINFÜHRHILFE FÜR STIMMPROTHESEN**
INSERTION AID FOR VOICE PROSTHESES
DISPOSITIF D'AIDE À L'INSERTION D'UNE PROTHÈSE PHONATOIRE

(30) Priorität: 22.02.2017 DE 102017103623
(43) Veröffentlichungstag der Anmeldung: 02.10.2019
(73) Patentinhaber: Andreas Fahl Medizintechnik-Vertrieb GmbH, 51149 Köln (DE)
(72) Erfinder: FAHL, Andreas, 51107 Köln (DE)
(74) Vertreter: Geskes, Christoph
(86) Internationale Anmeldenummer: PCT/EP2018/054333
(87) Internationale Veröffentlichungsnummer: WO 2018/153952

(56) Entgegenhaltungen:
- DE-T2- 69 607 767
- DE-U1-202008 000 670
- US-A- 5 064 433
- US-A- 5 935 165
- US-A1- 2009 043 386
- US-A1- 2011 093 071

## Beschreibung

Die vorliegende Erfindung betrifft eine Einführhilfe für eine Stimmprothese, umfassend eine stabförmige Vorrichtung und weiterhin eine Langhülse, als auch deren Verwendung.

Stimmprothesen dienen der Wiederherstellung der Stimme während oder nach einer Laryngektomie. Die Wiederherstellung der Stimme kann durch chirurgische Maßnahmen erfolgen. Wichtigstes Hilfsmittel dabei ist die Stimmprothese, die auch als "Shunt-Ventil" bezeichnet wird. Mittels einer Stimmprothese können Funktionen des entfernten Kehlkopfes ersetzt werden. Zum einen wird durch den Einsatz einer Stimmprothese eine Luftzufuhr von der Lunge über die Speiseröhre (Ösophagus) in den Rachen ermöglicht. Die Ausatemluft wird dabei zum Sprechen verwendet. Zum anderen dichtet die Stimmprothese die Verbindung von der Speiseröhre (Trachea) zur Luftröhre beim Schlucken von Speisen und Getränken ab, wodurch der Nutzer der Stimmprothese vor einem versehentlichen Verschlucken der Nahrung geschützt ist.

Ist die Stimmprothese als Verweilprothese ausgebildet, wird diese in aller Regel vom Arzt eingesetzt und/oder gewechselt. Solche Verweilprothesen werden in die sogenannte tracheosophageale Fistel, eine chirurgisch geschaffene Öffnung zwischen Luft- und Speiseröhre, eingesetzt. Eine Stimmbildung erfolgt durch Verschluss des Tracheostomas mit einem Finger und gleichzeitigem Ausatmen. Dabei öffnet sich das Ventil in der Stimmprothese durch den dabei entstehenden Druck und lässt Luft in die Speiseröhre ein, so dass ein Ton für die Sprache gebildet wird.

Derartige als Verweilprothesen ausgebildete Stimmprothesen weisen zwei Flansche auf, zwischen denen ein Schaft ausgebildet ist. Eine jede dem Schaft zugewandte Innenseite der Flansche der mehr oder weniger rotationssymmetrisch ausgebildeten Stimmprothese liegt an einer Oberfläche der Trachea bzw. einer Oberfläche des Ösophagus an. Der Schaft einer solchen Stimmprothese hingegen wird in der chirurgisch geschaffenen Öffnung, der tracheoösophagealen Fistel, angeordnet. Da die Flansche einen größeren Durchmesser als der Schaft aufweisen, gestaltet sich das Einsetzen solcher als Verweilprothesen ausgebildeter Stimmprothesen als schwierig. Eine Einführhilfe zur Einsetzung einer solchen Stimmprothese in die tracheoösophageale Fistel ist in DE 696 07 767 T2 offenbart. Dabei wird eine wie vorstehend beschriebene Stimmprothese an einem Einsetzelement angeordnet, indem diese auf einen zylindrischen Kopf an einem Ende des Einsetzelementes aufgesetzt wird. Der zylindrische Kopf ist dabei leicht konisch ausgebildet. Mittels eines Befestigungsnippels, der an einem Flansch, der an einer Wandung des Ösophagus zu liegen kommt, angeordnet ist, wird die Stimmprothese durch eine schlüssellochartige Öffnung, in welche der Befestigungsnippel eingeführt ist, sicher an dem Einsetzelement gehalten. Problematisch an dieser Einführhilfe ist, dass die Stimmprothese auf dem Kopf des Einführelementes locker aufsitzt, so dass bei der Anordnung des Befestigungsnippels in der schlüssellochartigen Öffnung, bei welcher es zu einer Dehnung des in aller Regel aus medizinischem Silikon gebildeten Materiales des Befestigungsnippels kommt, die Stimmprothese verrutschen oder auch wieder sich vom Kopfbereich lösen kann. Auch mag es möglich sein, dass die Stimmprothese hierbei beschädigt und damit unbrauchbar wird.

Aus dem Stand der Technik sind noch anders ausgestaltete Stimmprothesen als diejenigen der DE 696 07 767 T2 bekannt. So sind beispielsweise solche bekannt, welche ebenfalls einen Befestigungsnippel aufweisen, wobei dieser jedoch nicht durch eine schlüssellochartige Öffnung eines Einführelementes gehalten wird, sondern auf einem Einführelement, welches stabförmig ausgebildet ist, nach Aufsetzen der Stimmprothese auf einem zylinderförmigen Haltebereich an einem Ende des Einführelementes mit dem Einführelement verbunden wird, in dem der Befestigungsnippel mit einem in diesem befindlichen Loch auf einem Vorsprung am Einführelement befestigt wird. Aber auch hier erfolgt wiederum nur eine lose Anordnung der Stimmprothese auf dem zylinderförmigen Endbereich des Einsetzelementes, so dass es ebenfalls zu einem Verkanten, Abrutschen oder gar einer Beschädigung der Stimmprothese kommen kann.

US 5,064,433 A beschreibt eine Stimmprothese mit einer Einführhilfe, wobei die Sprachprothese ein Befestigungsmittel aufweist und die Einführhilfe ein integral und homogen darauf geformtes Befestigungsmittel aufweist, um die Vorrichtung lösbar mit dem Werkzeug zu verbinden.

US 2011/093071 A1 beschreibt ein Verfahren zum Einführen einer Stimmprothese mittels einer Gelkappe.

US 5,935,165 A beschreibt eine Einführhilfe mit einer aufspreitzbaren Aufnahme für eine Stimmprothese.

US 2009/043386 A1 beschreibt eine Stimmprothese und eine Einführhilfe die aufeinander angepasst sind.

DE 20 2008 000 670 U1 beschreibt eine Einführungsvorrichtung mit einem Führungsdrahtkanal.

Da grundsätzlich aus dem Stand der Technik eine Reihe unterschiedlicher Stimmprothesen bekannt sind, für welche jeweils ein spezifisches Einführelement zur Verfügung gestellt ist, kommt es für die Anwender zu der nicht komfortablen Situation, dass bei Verwendung unterschiedlicher Stimmprothesen stets unterschiedliche Einführelemente vorgehalten werden müssen.

Die vorliegende Erfindung stellt sich die Aufgabe, eine Einführhilfe für Stimmprothesen zur Verfügung zu stellen, die universal, d. h. unabhängig von der Ausgestaltung der aus dem Stand der Technik bekannten Stimmprothesen, eingesetzt werden kann und eine sichere Haltung der Stimmprothese auf dem Hilfsmittel ermöglicht.

Diese Aufgabe wird erfindungsgemäß gelöst durch eine Einführhilfe für eine Stimmprothese, umfassend eine stabförmige Vorrichtung mit einem an einem ersten Ende eines Stabes angeordneten Kopfbereich, dieser umfassend ein Anschlagelement für eine Stimmprothese und ein sich an dieses anschließendes, als ein auf dem Kopf stehender Kegelstumpf ausgebildetes Arretierungselement für eine Stimmprothese, wobei eine Kopffläche des als Kegelstumpf ausgebildeten Arretierungselementes mit dem größeren Durchmesser dem Anschlagelement abgewandt angeordnet ist, und wobei zwischen diesem Anschlagelement und dem Arretierungselement eine erste Ringfläche gebildet ist, die radial zu einer Längsachse des Stabes sich erstreckend ausgebildet ist. Die erfindungsgemäße Einführhilfe bietet den großen Vorteil, dass eine Stimmprothese, die an der stabförmigen Vorrichtung angeordnet ist, von dieser sicher, nämlich in einer arretierten, d. h. fixierten Position im Kopfbereich der stabförmigen Vorrichtung gehalten ist. Hierdurch ist ein Verrutschen oder Verwackeln und auch eine Beschädigung einer Stimmprothese nicht möglich. Der Bereich einer Stimmprothese, mit welcher das Arretierungselement in Kontakt kommt, ist in der Regel aus einem flexiblen, elastischen Material, wie medizinischem Silikon, gebildet. Das Arretierungselement der stabförmigen Vorrichtung tritt bei Aufsetzen einer Stimmprothese auf den Kopfbereich des Stabes mit einer Innenwandung der Stimmprothese in Kontakt und dehnt diese ggf. geringfügig auf. Diese Aufdehnungsfunktion des Arretierungselementes erfolgt über eine sehr kleine Fläche desselben, die mit der Innenwandung der Stimmprothese in Kontakt kommt, um im Übrigen diese nicht zu beschädigen als auch nach erfolgter Einsetzung in die tracheoösophageale Fistel ein leichtes Entfernen der stabförmigen Vorrichtung aus der eingesetzten Stimmprothese zu ermöglichen. Würde hier eine große Fläche zur Verfügung gestellt, würde sich die Entfernung der stabförmigen Vorrichtung schwierig gestalten oder aber zu unangenehmen Situationen wie Schmerzempfinden o. ä. bei der laryngektomierten Person führen.

Das Anschlagelement dient zusammen mit der ersten Ringfläche einer ausreichenden Abstützung einer Stimmprothese nach Anordnung derselben im Kopfbereich des Stabes der stabförmigen Vorrichtung, wobei eine Wandung eines Flansches einer Stimmprothese mit der ersten Ringfläche in Kontakt tritt oder treten kann. Hierdurch erfolgt einerseits eine Stabilisierung der aus einem flexiblen, elastischen Material hergestellten Stimmprothese durch Unterstützung dieses Flansches und des weiteren wird durch die Vorsehung des Anschlagelementes und der ersten Ringfläche verhindert, dass das Arretierungselement im Inneren der Stimmprothese nach Aufsetzen derselben zu weit geführt wird und das eigentliche Ventil im Inneren der Stimmprothese beschädigt. Die Flanschseite der Stimmprothese, die an die erste Ringfläche anlegbar ist, weist nach Einsatz derselben in der tracheoösophagealen Fistel in das Innere der Speiseröhre, steht also nicht in Kontakt mit der Oberfläche derselben.

Der Stab der stabförmigen Vorrichtung kann beispielsweise rund ausgebildet sein, ist bevorzugt jedoch abgeflacht ausgebildet mit einer im Wesentlichen ebenen, flachen Ober- und Unterseite, die miteinander über einen gerundeten Übergang verbunden sind. Der Stab weist mindestens eine, weiter bevorzugt mindestens zwei, noch weiter bevorzugt mindestens drei Befestigungsvorrichtungen für einen Befestigungsnippel an einer Stimmprothese auf. Die Befestigungsvorrichtungen können dabei vorteilhafterweise schlüssellochartig ausgebildet sein. Ein schlüssellochartiges Befestigungselement umfasst einen kreisrunden Öffnungsbereich und einen sich hieran anschließenden schlitzförmigen Öffnungsbereich. Ein Befestigungsnippel einer Stimmprothese kann durch den runden Öffnungsbereich geführt und anschießend im schlitzförmigen Öffnungsbereich des schlüssellochartigen Befestigungselementes fixiert werden. Bevorzugt umfasst der Stab der stabförmigen Vorrichtung mindestens zwei schlüssellochartig ausgebildete Befestigungselemente dieser Art. Weiter bevorzugt umfasst der Stab der stabförmigen Vorrichtung ein weiteres Befestigungselement, ausgebildet als Vorsprung. Dieser Vorsprung ist vorteilhafterweise auf einer ebenen, flachen Oberseite des Stabes angeordnet. Dieser Vorsprung ist vorteilhafterweise in seinem Querschnitt rund ausgebildet und weist weiter vorteilhafterweise die Form eines Zylinders auf. Ein solches Befestigungselement dient dazu, Befestigungsnippel von Stimmprothesen am Stab der stabförmigen Vorrichtung zu befestigen, bei welchen im Befestigungsnippel eine Öffnung ausgebildet ist, durch welche ein solches Befestigungsmittel führbar ist.

In einer besonders bevorzugten Ausführungsform sind am Stab der stabförmigen Vorrichtung der erfindungsgemäßen Einführhilfe genau drei Befestigungsmittel für einen Befestigungsnippel einer Stimmprothese vorgesehen, die der Befestigung unterschiedlicher, aus dem Stand der Technik bekannter Stimmprothesen ermöglichen. Dabei ist ausgehend vom Kopfbereich, der an dem ersten Ende des Stabes angeordnet ist, diesem als nächstes angeordnet bevorzugt ein erstes schlüssellochförmiges Befestigungselement. Nachfolgend diesem ist, bevorzugt angeordnet auf einer ebenen, flachen Oberseite des Stabes, wobei aber auch beispielsweise eine entsprechende Anordnung bei einer runden Ausbildung des Stabes möglich ist, ein zylinderförmiges Befestigungselement. Diesem nachfolgend in Richtung auf ein dem ersten Ende des Stabes entgegengesetztes zweites Ende desselben ist ein zweites schlüssellochförmiges Befestigungselement angeordnet.

Das zweite Ende des Stabes weist vorteilhafterweise mindestens ein Greifelement, bevorzugt mindestens zwei Greifelemente auf. Das Greifelement ist bevorzugt muldenförmig im Stab der stabförmigen Vorrichtung ausgebildet. Bevorzugt weist das Greifelement eine Riffelung auf, um ein sicheres Halten mit einem Finger des Anwenders zu ermöglichen. Bevorzugt ist zumindest eine Teilfläche des Griffelementes mit einer solchen Riffelung versehen, weiter bevorzugt ist bei Ausbildung des Greifelementes mit einer Mulde die Mulde zumindest teilflächig, weiter bevorzugt vollflächig mit einer solchen Riffelung versehen. In einer besonders bevorzugten Ausführungsform sind genau zwei aneinander angrenzende Greifelemente am zweiten Ende des Stabes der stabförmigen Vorrichtung angeordnet, wobei weiter bevorzugt diese beiden Greifelemente muldenförmig ausgebildet sind, und weiter bevorzugt über eine gesamte Fläche der Mulde eine Riffelung aufweisen.

Weiter bevorzugt umfasst der Stab der stabförmigen Vorrichtung mindestens ein Markierungsmittel, bevorzugt mindestens zwei Markierungsmittel. Diese Markierungsmittel sind bevorzugt benachbart dem mindestens einen Greifelement angeordnet. Bevorzugt umfasst das Markierungsmittel auf einer Oberfläche des Stabes der stabförmigen Vorrichtung einen Vorsprung, weiter bevorzugt einen linienförmigen Vorsprung, der in einer Ebene senkrecht zu der Längsachse des Stabes auf der Oberfläche desselben angeordnet ist. Bevorzugt ist dabei eine Ausgestaltung, bei der der linienförmige Vorsprung des Markierungsmittels auf einer ebenen, flachen Oberseite des Stabes angeordnet ist. Das Markierungselement dient dem Anwender insbesondere dazu, eine Information zu erhalten, an welcher Stelle sich eine an der erfindungsgemäßen Einführhilfe angeordnete Stimmprothese befindet. Diese umfasst neben der stabförmigen Vorrichtung vorzugsweise weiterhin mindestens eine Langhülse. Nach Anordnung der Stimmprothese auf der stabförmigen Vorrichtung der erfindungsgemäßen Einführhilfe wird die stabförmige Vorrichtung mit der Stimmprothese in die Langhülse eingeführt. Über einen Ausführendbereich, der sich verjüngend im Vergleich zu einem Einführendbereich der Langhülse ausgebildet ist, wird dann die Stimmprothese gezielt in der tracheoösophagealen Fistel platziert und in diese eingesetzt. Zur Ermittlung der Anordnung der Stimmprothese in diesem Ausführendbereich der Langhülse dient das mindestens eine Markierungselement.

In einer bevorzugten Ausführungsform ist die im Kopfbereich des Stabes der stabförmigen Vorrichtung angeordnete erste Ringfläche durch das Anschlagelement gebildet. Das Anschlagelement ist das dem ersten Ende des Stabes zugewandte erste Element des Kopfbereiches und ist weiter bevorzugt unmittelbar am ersten Ende des Stabes der stabförmigen Vorrichtung angeordnet. Bevorzugt ist das Anschlagelement zylinderförmig ausgebildet. Es weist bevorzugt eine rotationssymmetrischen Ausbildung auf. Eine Mantelumfangsfläche des Anschlagelementes überragt dabei die Dimension des Stabes an sich in zumindest eine Richtung, bevorzugt in sämtliche Richtungen. Die erste Ringfläche wird mithin durch eine Kopffläche des zylinderförmigen Anschlagelementes gebildet.

Erfindungsgemäß ist das Arretierungselement kegelstumpfförmig ausgebildet. Das Arretierungselement ist vorzugsweise rotationssymmetrisch ausgebildet, es weist vorteilhafterweise einen geringeren Radius bzw. Durchmesser auf als das Anschlagelement. Eine Außenwandung des Arretierungselementes ist vorteilhafterweise geneigt auf die erste Ringfläche zu, ausgehend von einer Kopffläche des Arretierungselementes, ausgebildet. Die Kopffläche des Arretierungselementes wiederum bildet bevorzugt eine zweite Ringfläche. Mithin umfasst das Arretierungselement bevorzugt eine zweite Ringfläche. Diese zweite Ringfläche ist bevorzugt beabstandet von der ersten Ringfläche angeordnet. Bevorzugt ist das kegelstumpfförmige Arretierungselement derart im Kopfbereich angeordnet, dass die Endfläche geringeren Durchmessers verbunden ist mit einer Oberseite des Anschlagelementes, durch welches die erste Ringfläche gebildet ist. Demgegenüber ist die Kopffläche des kegelstumpfförmigen Arretierungselementes mit dem größeren Durchmesser dem Anschlagelement abgewandt angeordnet. Mithin kann die Ausgestaltung des erfindungsgemäßen Arretierungselementes der Form nach beschrieben werden als ein auf dem Kopf stehender Kegelstumpf, verbunden mit dem Anschlagelement.

In einer weiter bevorzugten Ausführungsform ist ein Übergang zwischen der zweiten Ringfläche und der Außenfläche des Arretierungselementes als Kante ausgebildet. Die Kante kann geringfügig gerundet ausgebildet sein. Diese Kante kommt bei Anordnung einer Stimmprothese mit einer Innenwandung des Schaftes derselben in Kontakt, und liegt dabei punktuell über die durch die Kante gebildete Umfangslinie den Schaft auf. Hierdurch erfolgt eine sichere Arretierung bei gleichzeitig leichter Entfernbarkeit der stabförmigen Vorrichtung nach Einsetzen einer Stimmprothese in der tracheoösophagealen Fistel.

Weiter bevorzugt ist ein Winkel α zwischen der Außenfläche des Arretierungselementes und der zweiten Ringfläche in einem Bereich von etwa 50° bis etwa 86° gebildet, weiter bevorzugt in einem Bereich von etwa 65° bis etwa 85°, noch weiter bevorzugt in einem Bereich von etwa 72° bis etwa 83°. Durch diese Ausgestaltung des Arretierungselementes ist bei Anordnung einer Stimmprothese auf den Kopfbereich der stabförmigen Vorrichtung sichergestellt, dass zwischen der Außenfläche und einer Innenwandung des Schaftes einer Stimmprothese ausreichend Platz geschaffen ist, so dass ein einfaches, insbesondere auch schmerzfreies, Entfernen der Einführhilfe nach Einsatz der Stimmprothese in die tracheoösophagealen Fistel sichergestellt ist.

Weiter bevorzugt umfasst der Kopfbereich ein Vorarretierungsmittel. Weiter bevorzugt ist das Vorarretierungsmittel benachbart, bevorzugt unmittelbar benachbart, dem Arretierungsmittel angeordnet. Bevorzugt ist das Vorarretierungsmittel zylinderstabförmig ausgebildet, ebenso wie auch das Anschlagelement zylinderstabförmig ausgebildet sein kann. Bevorzugt ist ein Durchmesser des rotationssymmetrisch ausgebildeten Vorarretierungselementes kleiner als derjenige des Arretierungselementes. Weiter bevorzugt ist ein Durchmesser des Vorarretierungsmittels auch kleiner als ein durch die Kante des Arretierungselementes definierter Durchmesser desselben. Weiter bevorzugt weist das Vorarretierungselement eine größere Höhe auf als das Anschlagelement. Das Vorarretierungselement dient einer Vorhaltung bei Anordnung einer Stimmprothese auf der stabförmigen Vorrichtung, wodurch insbesondere der Arretierungsschritt der Stimmprothese für den Anwender erleichtert ist. Das Vorarretierungselement ist dabei derart dimensioniert, dass es nicht zu einer Arretierung oder Fixierung im Schaft einer Stimmprothese kommt, sondern dass lediglich die Stimmprothese locker mit ihrem Schaft auf dem Vorarretierungselement aufgesetzt ist.

In einer bevorzugten Ausgestaltung ist das Anschlagelement und/oder das Vorarretierungselement zu dem Stab und der durch diesen verlaufenden Längsachse koaxial ausgebildet, wobei weiter bevorzugt eine Mittelachse des Anschlagelementes und/oder des Vorarretierungselementes mit der Längsachse des Stabes zusammenfällt. Weiter bevorzugt ist auch das Arretierungselement ausgebildet wie das Anschlagelement und/oder das Vorarretierungselement wie vorstehend beschrieben.

In einer besonders bevorzugten Ausgestaltung ist der Kopfbereich der stabförmigen Vorrichtung gebildet aus einem zylinderförmigen Anschlagelement, einem unmittelbar an diesem sich anschließenden kegelstumpfförmigen Arretierungselement und einem sich unmittelbar an diesem anschließenden Vorarretierungselement, welches zylinderförmig ausgebildet ist. Dabei ist vorzugsweise ein Durchmesser des Arretierungselementes größer als ein Durchmesser, definiert durch die Kante des Arretierungselementes, und dieser wiederum größer als ein Durchmesser des zylinderförmigen Vorarretierungselementes. Bevorzugt ist ein Durchmesser der ersten Ringfläche größer als ein Durchmesser der zweiten Ringfläche. Bevorzugt wird die erste Ringfläche dabei gebildet von dem Anschlagelement und die zweite Ringfläche gebildet von dem Arretierungselement.

In einer bevorzugten Ausführungsform ist unterhalb des Kopfbereiches am Stab mindestens eine Materialanhäufung, bevorzugt mindestens zwei Materialanhäufungen angeordnet. Diese Materialanhäufung ist vorzugsweise dann ausgebildet, wenn der Stab nicht rund ausgebildet ist, sondern insbesondere eine ebene, abgeflachte Oberseite und Unterseite aufweist, welche bevorzugt durch einen gerundeten Bereich miteinander verbunden sind. Ausgehend von der ebenen, flachen Oberseite bzw. Unterseite des Stabes und sich in ihrer Dicke verstärkend ist die Materialanhäufung auf dem Stab angeordnet. Bevorzugt erstreckt sich die Materialanhäufung bis zu einer Unterfläche des Anschlagelementes und grenzt bevorzugt unmittelbar an diese. Durch die Vorsehung der mindestens einen Materialanhäufung ist dem Stab der stabförmigen Vorrichtung in Hinblick auf die Ausgestaltung des Kopfbereiches eine hinreichende Stabilität mitgegeben, so dass ein sicheres Einsetzen einer Stimmprothese erreichbar ist.

Die erfindungsgemäße Einführhilfe umfasst weiter vorteilhafterweise eine Langhülse. In diese Langhülse ist vorteilhafterweise die stabförmige Vorrichtung einführbar. Die Einführung der stabförmigen Vorrichtung erfolgt dabei zum Zwecke des Einsatzes einer Stimmprothese nach Anordnung einer Stimmprothese im Kopfbereich der stabförmigen Vorrichtung. Die Langhülse dient dazu, dem Anwender, beispielsweise dem Arzt oder dem Patienten, bei Einsetzten der Stimmprothese eine sichere Führung mitzugeben. Vorteilhafterweise ist die Langhülse in einem Ausführendbereich elastisch ausgebildet. Durch diesen Ausführendbereich erfolgt letztendlich nach Durchführung der Stimmprothese deren Einsatz in der tracheoösophagealen Fistel. Der Ausführendbereich ist dabei stärker konisch ausgebildet als eine diesem vorgelagerte Wandung der Langhülse, die ebenfalls leicht konisch ausgebildet ist. Durch die stärkere konische Ausbildung kommt es zu einer stärkeren Einfaltung der Flansche der Stimmprothese vor Ausführung durch die Öffnung im Ausführendbereich der Langhülse, so dass durch die chirurgisch angelegte Öffnung, die kleiner ist als ein Flansch einer Stimmprothese, die Stimmprothese gleichwohl durchführbar ist. Die elastische Ausbildung kann beispielsweise dadurch erfolgen, dass mindestens zwei, bevorzugt mindestens drei, weiter bevorzugt mindestens vier Schlitze, in Richtung einer Längsachse der Längshülse, im Ausführendbereich der Langhülse vorgesehen sind. Hierdurch sind diejenigen zwischen den Schlitzen, welche koaxial zu einer Längsachse der Langhülse angeordnet sind, liegenden Bereiche elastisch beweglich und können sich an die Bauform der einzusetzenden Stimmprothese etwas anpassen. Bevorzugt wird eine elastische Ausbildung im Ausführendbereich auch erreicht durch Vorsehung eines zumindest geringelastischen Materiales für die Langhülse. Bevorzugt ist die Langhülse in ihrem Ausführendbereich aus einem zumindest geringelastischen Material ausgebildet und weist weiterhin die vorstehend genannten Langschlitze auf. Besonders bevorzugt weist der Ausführendbereich genau drei bzw. vier Langschlitze auf, die jeweils in einem Winkel um 120° bzw. 90° um eine Umfangsrichtung des Ausführendbereiches versetzt an diesem angeordnet sind. Im Bereich der Öffnung im Ausführendbereich ist diese abgerundet ausgebildet, um eine Verletzung bei Einsatz der Stimmprothese in die tracheoösophageale Fistel zu vermeiden.

Besonders bevorzugt ist in einem dem Ausführendbereich entgegengesetzten Einführendbereich der Langhülse, in welchem die Stimmprothese mittels der stabförmigen Vorrichtung einführbar ist, ein Aufnahmeschlitz ausgebildet. Dieser ermöglicht eine gezielte Faltung eines, bevorzugt beider Flansche einer in die Langhülse über die stabförmige Vorrichtung einzuführenden Stimmprothese. Bevorzugt ist der Einführendbereich trichterförmig ausgebildet, wodurch die Aufnahme und Führung einer Stimmprothese, angeordnet an einer stabförmigen Vorrichtung, erleichtert ist. Besonders bevorzugt ist die Wandung der Langhülse konisch vom Einführendbereich auf den Ausführendbereich hin zulaufend ausgebildet. Wie bereits vorstehend beschrieben, ist der Ausführendbereich selbst stärker konisch ausgebildet als die konische Ausbildung der Wandung zwischen Ausführendbereich und Einführendbereich. Die leichte konische Ausbildung der Wandung der Langhülse im Bereich zwischen Einführendbereich und Ausführendbereich sorgt für eine Vorkonfiguration der einzuführenden Stimmprothese vor Ausführung über den Ausführendbereich in die tracheoösophageale Fistel.

Die vorliegende Erfindung betrifft weiterhin eine stabförmige Vorrichtung für eine Stimmprothese, wie vorstehend im Zusammenhang mit der erfindungsgemäßen Einführhilfe beschrieben. Diesbezüglich sei auf die vorstehenden Ausführungen hingewiesen. Die erfindungsgemäße Einführhilfe und die stabförmige Vorrichtung, wie vorstehend beschrieben, wird zur Einsetzung einer Stimmprothese in eine Öffnung zwischen Trachea und Ösophagus, mithin in eine tracheoösophageale Fistel, verwendet.

Diese und weitere Vorteile der vorliegenden Erfindung werden anhand der nachfolgenden Figuren näher erläutert. Es zeigen:
- Fig. 1:: eine erfindungsgemäße stabförmige Vorrichtung in einer Draufsicht;
- Fig. 2:: die stabförmige Vorrichtung gemäß Fig. 1 in einer perspektivischen Ansicht;
- Fig. 3:: die stabförmige Vorrichtung gemäß Fig. 1 in einer Seitenansicht;
- Fig. 4:: den Kopfbereich der Vorrichtung gemäß Fig. 1 in einer vergrößerten Ansicht;
- Fig. 5:: eine Langhülse der erfindungsgemäßen Einführhilfe in einer perspektivischen Ansicht;
- Fig. 6:: die Langhülse gemäß Fig. 5 in einer Draufsicht;
- Fig. 7:: die Einführhilfe gemäß Fig. 5 in einem Schnitt;
- Fig. 8:: die Einführhilfe gemäß Fig. 5 in einer vergrößerten Vorderansicht;
- Fig. 9:: drei Zustände einer Anordnung einer Stimmprothese auf der stabförmigen Vorrichtung gemäß Fig. 1;
- Fig. 10:: eine Einzelheit einer Anordnungsstellung einer Stimmprothese auf der stabförmigen Vorrichtung gemäß Fig.1 (Vorarretierungsstellung);
- Fig. 11:: eine zweite Anordnungsstellung einer Stimmprothese auf der stabförmigen Vorrichtung gemäß Fig. 1 (Arretierungs- und Anschlagsstellung);
- Fig. 12:: eine Teilansicht einer erfindungsgemäßen Einführhilfe umfassend eine stabförmige Vorrichtung und eine Langhülse, wobei die stabförmige Vorrichtung eine Stimmprothese trägt; und
- Fig. 13:: eine Schnittansicht einer Anordnungsstellung einer Stimmprothese auf der stabförmigen Vorrichtung gemäß Fig. 1 (Arretierungs- und Anschlagstellung).

Zunächst sei vorausgeschickt, dass die in den Figuren dargestellten Ausführungsformen nicht beschränkend auszulegen sind. Vielmehr können die dort beschriebenen Merkmale untereinander und mit den zuvor beschriebenen Merkmalen zur weiteren Ausgestaltung kombiniert werden. So können beispielsweise auch nur zwei Befestigungselemente für einen Befestigungsnippel einer Stimmprothese vorgesehen sein, oder aber auch mehr als drei solcher Befestigungselemente. Diese können untereinander auch unterschiedlich ausgebildet sein, beispielsweise in Form von schlüssellochförmigen Befestigungselementen oder aber zylinderförmigen Vorsprüngen. Zudem kann auch nur eine Griffmulde für ein Griffelement vorgesehen sein und auch nur ein Markierungselement. Auch kann ein Ausführendbereich einer Langhülse mit mehr oder weniger Schlitzen oder aber auch ohne Schlitze bei einer hinreichend elastischen Ausbildung des Ausführendbereiches ausgebildet sein.

Schließlich sei darauf hingewiesen, dass die in der Figurenbeschreibung und den Ansprüchen angegebenen Bezugszeichen den Schutz der vorliegenden Erfindung nicht beschränken, sondern lediglich auf die in den Figuren gezeigten Ausführungsformen verweisen.

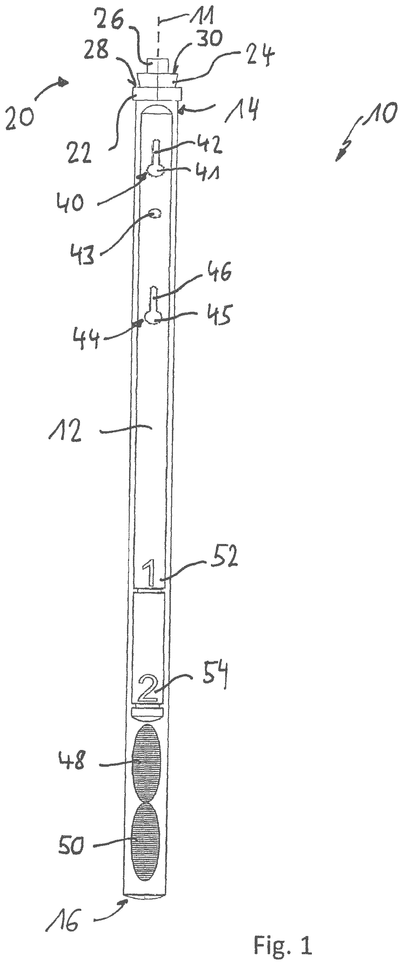

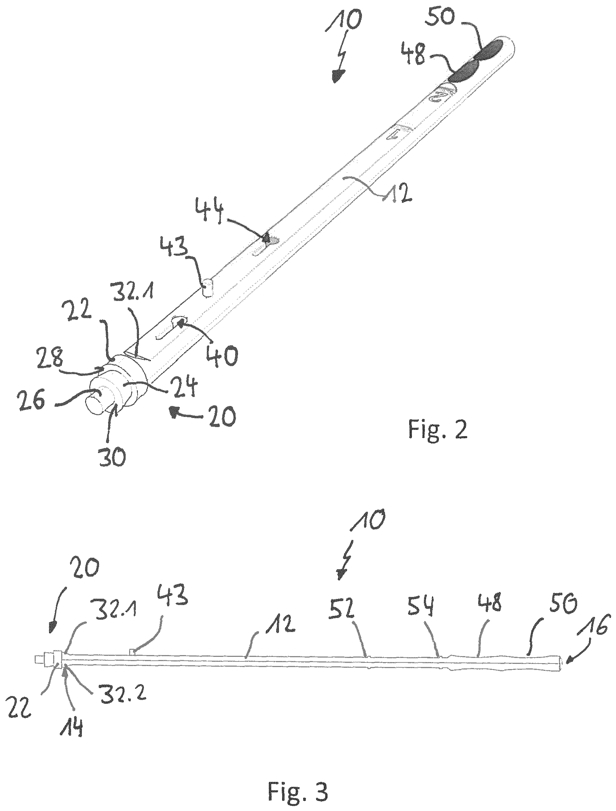

Fig. 1 zeigt eine insgesamt mit dem Bezugszeichen 10 bezeichnete erfindungsgemäße stabförmige Vorrichtung, die Bestandteil einer erfindungsgemäßen Einführhilfe sein kann, in einer Draufsicht. Die stabförmige Vorrichtung 10 weist einen Stab 12 mit einem ersten Ende 14 und einem zweiten Ende 16 auf. Der Stab 12 weist nahe dem zweiten Ende 16 zwei muldenförmige Griffelemente 48 und 50 für Finger des Anwenders auf, wobei die Mulden mehr oder weniger vollflächig mit einer Riffelung versehen sind. Der Stab 12 weist weiterhin zwei Markierungselemente 52 und 54 auf, deren Ausgestaltung nähergehend zu Fig. 3 unten erläutert ist. Weiterhin weist der Stab 12 drei Befestigungselemente 40, 43 und 44 auf. Die Befestigungselemente 40 und 44 sind schlüssellochförmig ausgebildet mit jeweils einer runden Öffnung 41 bzw. 45 und einer schlitzförmigen, sich an die runde Öffnung anschließenden Öffnung 42 bzw. 46. Zwischen den Befestigungselementen 40 und 44 ist ein weiteres Befestigungselement 43, ausgebildet als zylinderförmiger Vorsprung (siehe hierzu Fig. 2 und 3), angeordnet.

Unmittelbar am ersten Ende 14 angeordnet ist ein Kopfbereich 20 der stabförmigen Vorrichtung 10 mit einem Anschlagelement 22, einem diesem unmittelbar benachbart angeordneten Arretierungselement 24 und einem diesem unmittelbar benachbart angeordneten Vorarretierungselement 26. Das Anschlagelement 22, das Arretierungselement 24 und das Vorarretierungselement 26 sind dabei rotationssymmetrisch ausgebildet und koaxial zu einer Längsachse 11 des Stabes 12, wobei die Mittelachsen des Anschlagelementes 22, des Arretierungselementes 24 und des Vorarretierungselementes 26 mit der Längsachse 11 des Stabes 12 zusammenfallen.

Das Anschlagelement 22 bildet eine erste Ringfläche 28 und das Arretierungselement 24 eine zweite Ringfläche 30. Der Durchmesser des Anschlagelementes 22 ist größer als der maximale Durchmesser des Arretierungselementes 24, der wiederum größer ist als der Durchmesser des Vorarretierungselementes. Das Anschlagelement 22 und das Vorarretierungselement 26 sind zylinderförmig ausgebildet. Das Arretierungselement 24 ist als umgekehrter Kegelstumpf ausgebildet.

Fig. 2 zeigt die stabförmige Vorrichtung 10 gemäß Fig. 1 in einer perspektivischen Ansicht, auf welcher insbesondere gut die Ausbildung des weiteren, als zylinderförmigen Vorsprung ausgebildeten Befestigungselementes 43 zu erkennen ist. Auch ist gut die Ausgestaltung einer ersten Materialanhäufung 32.1 zu erkennen. Der Stab 12 selbst ist abgeflacht ausgebildet mit einer ebenen, flachen Oberseite und einer dieser gegenüberliegend entsprechend ausgebildeten Unterseite, welche miteinander über einen gerundeten Bereich verbunden sind. Ausgehend von einer Oberseite des Stabes 12 ist die Materialanhäufung 32.1 in ihrer Materialstärke ansteigend zum Kopfbereich 20 der stabförmigen Vorrichtung 10 hin ausgebildet.

Fig. 3 ist eine Seitenansicht der stabförmigen Vorrichtung gemäß Fig. 1 und zeigt insbesondere die Ausgestaltung der ersten Materialanhäufung 32.1 und einer dieser gegenüberliegend auf einer Unterseite des Stabes 12 angeordneten zweiten Materialanhäufung 32.2. Insbesondere ist in Fig. 3 gut zu erkennen, dass die beiden Materialanhäufungen 32.1 und 32.2 unmittelbar an das Anschlagelement 22 angrenzen. Fig. 3 ist weiterhin zu entnehmen die Ausgestaltung der beiden Markierungselemente 52 und 54. Gut ist zu erkennen, dass diese als linienförmige Vorsprünge auf der Oberseite und der Unterseite des Stabes 12 angeordnet sind, wobei diese linienförmigen Vorsprünge in einer Ebene senkrecht zu der Längsachse 11 des Stabes 12 (siehe Fig. 1) verlaufen.

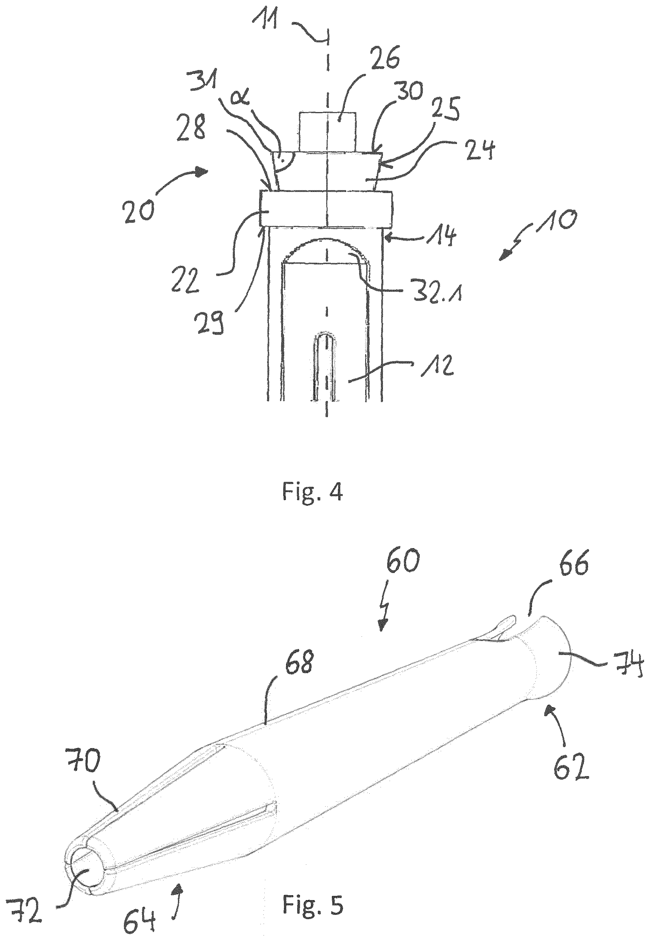

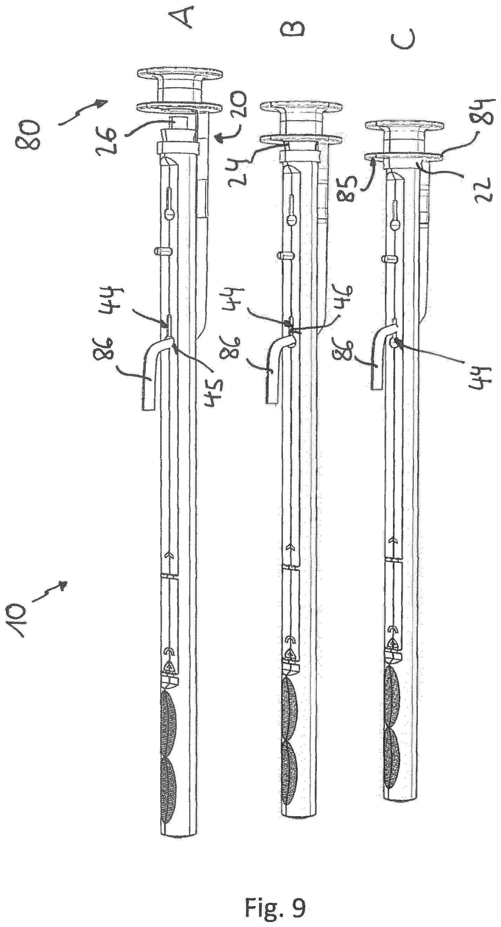

Fig. 4 zeigt eine Einzelheit der Ausgestaltung des Kopfbereiches 20 der stabförmigen Vorrichtung 10 gemäß Fig. 1. Die erste Materialanhäufung 32.1 am ersten Ende 14 des Stabes 12 grenzt unmittelbar an das Anschlagelement 22, genauer an eine Unterfläche 29 desselben, an. Das Arretierungselement 24 bildet zwischen der zweiten Ringfläche 30 und einer Außenfläche 25 desselben einen Winkel a, der bei etwa 80° liegt. Zwischen der zweiten Ringfläche 30 und der Außenfläche 25 des Arretierungselementes 24 ist eine Kante 31 gebildet, deren linienförmige Umfangsfläche bei Anordnung einer Stimmprothese 80 (s. Fig. 9 bis 13) auf der stabförmigen Vorrichtung 10 mit einer Innenwandung eines Schaftes einer solchen Stimmprothese 80 zusammenwirkt und dadurch diese arretiert. Die umgekehrt kegelstumpfförmige Ausgestaltung des Arretierungselementes 24 führt dazu, dass eine Arretierung einer Stimmprothese 80 letztendlich nur über den Bereich der Umfangslinie der Kante 31 des Arretierungselementes 24 erfolgt, so dass die stabförmige Vorrichtung 10 nach Einsetzen der Stimmprothese 80 schmerzfrei wieder entfernt werden kann.

Fig. 5 zeigt in einer perspektivischen Ansicht eine Langhülse 60, die hiermit ebenfalls zum Gegenstand der vorliegenden Anmeldung gemacht wird. Diese Langhülse 60 weist einen Ausführendbereich 64 und einen Einführendbereich 62 auf. Der Einführendbereich 62 weist einen Aufnahmeschlitz 66 auf. Weiterhin ist der Einführendbereich 62 trichterhaft mit einem Trichter 74 ausgebildet, wobei der Aufnahmeschlitz 66 im trichterhaften Teil 74 des Einführendbereiches 62 überwiegend angeordnet ist. Gegenüberliegend dem Einführendbereich 62 ist der Ausführendbereich 64 der Langhülse 60 ausgestaltet. Dieser weist eine stärkere konische Ausbildung auf als eine Wandung 68 im Bereich zwischen dem Einführendbereich 62 und dem Ausführendbereich 64, wobei die Wandung 68 gleichwohl auch leicht konisch ausgehend vom Einführendbereich 62 zum Ausführendbereich 64 ausgebildet ist. Der Ausführendbereich 64 weist insgesamt vier linienförmige Schlitze 70 auf und umfasst eine Ausführöffnung 62, wobei diese gerundet ausgebildet ist.

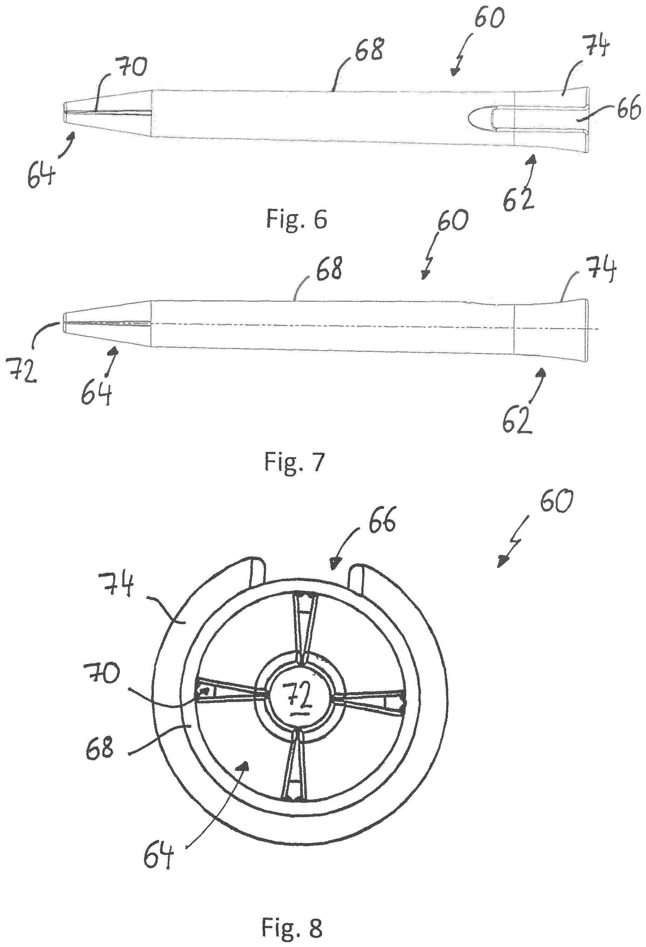

Fig. 6 zeigt zur Verdeutlichung die Langhülse 60 gemäß Fig. 5 in einer Draufsicht, wobei insbesondere gut die Ausbildung des Aufnahmeschlitzes 64 im Einführendbereich 62 im Bereich des Trichters 74 ersichtlich ist.

Fig. 7 hingegen, die einen Schnitt durch die Langhülse 60 darstellt, kann entnommen werden, dass die Wandung 68 leicht konisch vom Einführendbereich 62 auf den Ausführendbereich 64 zu ausgebildet ist und dabei über die gesamte Länge eine mehr oder weniger gleiche Materialstärke aufweist.

Fig. 8 zeigt schließlich in einer vergrößerten Frontalansicht die Langhülse 60 gemäß Fig. 5, wobei besonders gut die Ausbildung der vier Schlitze 70 und deren Verlauf auf die Öffnung 70 im Ausführendbereich 64 erkenntlich wird. Auch ist die leicht konische Ausbildung der Wandung 68 als auch der Trichter 74 zu erkennen.

Fig. 9 zeigt in einer Ausgangsstellung A die Anordnung einer Stimmprothese 80 auf der stabförmigen Vorrichtung 10, wobei die Stimmprothese 80 einen Befestigungsnippel 86 aufweist, der durch das schlüssellochförmige Befestigungselement 44 und dessen runde Öffnung 45 geführt ist. Die Stellung B zeigt eine Arretierungsstellung, in welcher die Stimmprothese 80 dem Arretierungselement 24 zugeordnet ist, und durch dieses bereits arretiert ist. Durch diese Arretierung, kann der Befestigungsnippel 86 der Stimmprothese 80 sicher im schlüssellochförmigen Befestigungsmittel 44 und insbesondere in dem schlitzförmigen Teil 46 desselben angeordnet werden. Stellung C zeigt die Anschlag- und Arretierungsstellung der Stimmprothese 80, wobei ein Flansch 84 mit seiner Außenfläche 85 unmittelbar an der ersten Ringfläche 28 des Anschlagelementes 22 zur Anlage kommt.

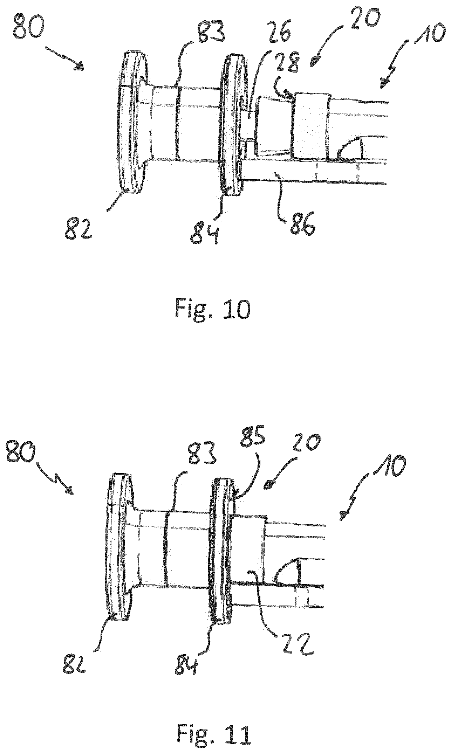

Die Fig. 10 und 11 zeigen eine Stellung zwischen A und B als auch die Stellung C gemäß Fig. 9 im Einzelnen. Insbesondere ist die Stimmprothese 80 nähergehend definiert als neben dem der stabförmigen Vorrichtung 10 zugewandten Flansch 84 einen Flansch 82 umfassend, wobei zwischen den beiden Flanschen 82 und 84 ein Schaft 83 angeordnet ist. Der Flansch 82 muss mittels der Längshülse und der stabförmigen Vorrichtung 10 gezielt gefaltet werden, um einen Durchtritt durch die von der tracheoösophagealen Fistel bereitgestellte Öffnung zu ermöglichen. Gut ist die Vorhaltung bzw. Vorarretierung durch das Vorarretierungsmittel 26 zu erkennen und insbesondere, dass dieses nicht mit einer Innenwandung des Schaftes 83 in Kontakt tritt. Fig. 11 zeigt die bereits vorstehend im Zusammenhang mit Fig. 9, Stellung C beschriebene Anschlags- und Arretierungsstellung mit dem Zusammenwirken von Anschlagelement 22 und Flansch 84 der Stimmprothese 80.

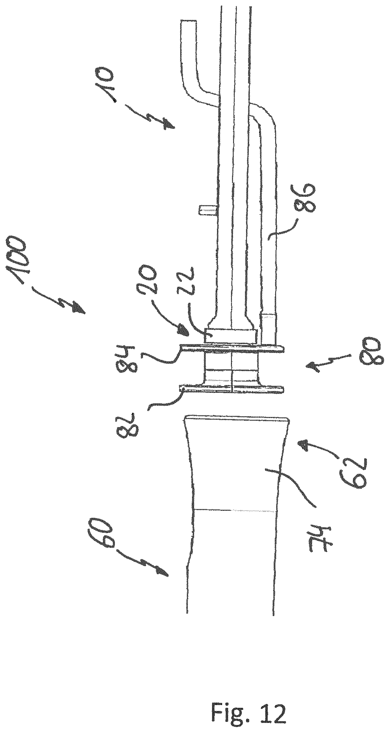

Fig. 12 zeigt die erfindungsgemäße Einführhilfe 100 mit der stabförmigen Vorrichtung 10 und der Langhülse 60. Die stabförmige Vorrichtung 10 weist dabei in Arretierungs- und Anschlagstellung eine Stimmprothese 80 auf, und zwar kurz vor Einführung in den Trichter 74 des Einführendbereiches 62 der Langhülse 60.

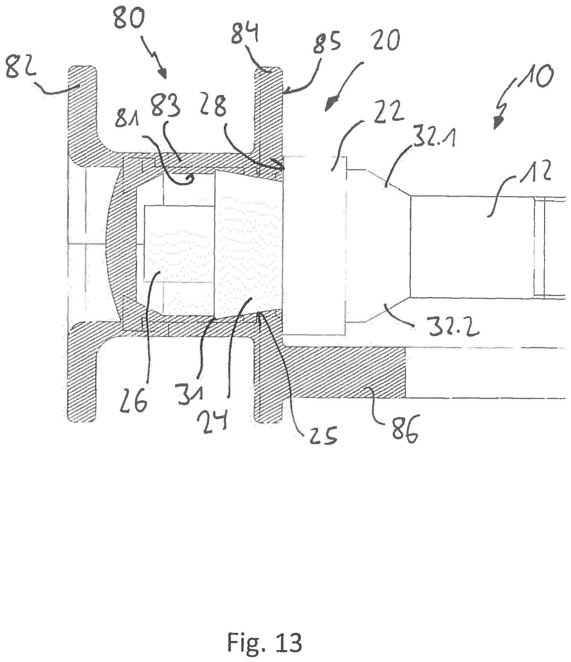

Fig. 13 zeigt in einem Schnitt die Anschlags- und Arretierungsstellung gemäß Fig. 11. Gut ist zu erkennen, dass die Kante 31 des Arretierungselementes 24 in das Material des Schaftes 83 eindringt bzw. den Schaft über die Umfangslinie der Kante 31 aufdehnt und hierüber eine Arretierung der Stimmprothese 80 durch die stabförmige Vorrichtung 10 erfolgt. Im Bereich der Außenwandung 25 erfolgt benachbart der Kante 31 durch das Arretierungselement 24 auch noch eine gewisse Haltung an der Innenwandung 81 des Schaftes 83. Fig. 13 stellt eine schematische Ansicht dar, die nicht ganz der tatsächlich erfolgten Arretierungsstellung entspricht. Denn das Material der Stimmprothese 80 und insbesondere deren Schaftes 83 ist relativ weich und elastisch, und in der Regel aus medizinischem Silikon gebildet. Die entsprechende Nachgiebigkeit des Schaftmateriales, insbesondere auch der Innenwandung 81 des Schaftes 83, ist in Fig. 13 nicht gezeigt. Hierdurch entsteht der Eindruck, dass im Bereich der Außenfläche 25 des Arretierungselementes 24 es zu einer umfangreichen Anlage an die Innenwandung 81 des Schaftes 83 kommt. Tatsächlich erfolgt jedoch maßgeblich mehr oder weniger nur ein linienförmiger Kontakt entlang der Umfangslinie der Kante 31 des Arretierungselementes 24. Gut ist in Fig. 13 auch zu erkennen der Kontakt zwischen der Außenseite 85 des Flansches 84 mit der ersten Ringfläche 28 des Anschlagelementes 22, als auch die Ausbildung der ersten und der zweiten Materialanhäufung 32.1 und 32.2.

Durch die vorliegende Erfindung ist damit eine universell einsetzbare Einführhilfe und eine universell einsetzbare stabförmige Vorrichtung zur Verfügung gestellt, mit welcher Stimmprothesen unterschiedlicher Ausgestaltung und unterschiedlicher Hersteller in die tracheoösophageale Fistel eingesetzt werden können. Insbesondere weist die stabförmige Vorrichtung vorteilhafterweise ein Arretierungselement auf, wodurch eine sichere und hinreichend feste Haltung der Stimmprothese bei Einsatz derselben, insbesondere unter Zuhilfenahme der Langhülse, in die tracheoösophageale Fistel gewährleistet ist. Insbesondere bei der Ausführung mit einem Arretierungselement im Kopfbereich der stabförmigen Vorrichtung, bei welcher nur ein mehr oder weniger linienförmiger Kontakt zwischen einer Umfangskante 31 und einer Innenwandung eines Schaftes einer Stimmprothese erfolgt, kann zudem neben einer sicheren Arretierung auch eine in aller Regel hoffentlich schmerzfreie Entfernung der Einführhilfe nach Einsetzen der Stimmprothese erfolgen.

## Patentansprüche

1. Einführhilfe (100) für eine Stimmprothese (80), umfassend eine stabförmige Vorrichtung (10) mit einem an einem ersten Ende (14) eines Stabes (12) angeordneten Kopfbereich (20), dieser umfassend ein Anschlagelement (22) für eine Stimmprothese (80) und ein sich an dieses anschließendes, als ein auf dem Kopf stehender Kegelstumpf ausgebildetes Arretierungselement (24) für eine Stimmprothese (80), wobei eine Kopffläche des als Kegelstumpf ausgebildeten Arretierungselementes (24) mit dem größeren Durchmesser dem Anschlagelement (22) abgewandt angeordnet ist, und wobei zwischen dem Anschlagelement (22) und dem Arretierungselement (24) eine erste Ringfläche (28) gebildet ist, die sich radial zu einer Längsachse (11) des Stabes (12) erstreckend ausgebildet ist.

2. Einführhilfe (100) gemäß Anspruch 1, **dadurch gekennzeichnet, dass** die erste Ringfläche (28) durch das Anschlagelement (22) gebildet ist.

3. Einführhilfe (100) gemäß einem oder mehreren der vorhergehenden Ansprüche, **dadurch gekennzeichnet, dass** das Arretierungselement (24) eine zweite Ringfläche (30) umfasst.

4. Einführhilfe (100) gemäß einem oder mehreren der vorhergehenden Ansprüche, **dadurch gekennzeichnet, dass** ein Übergang zwischen der zweiten Ringfläche (30) und einer Außenfläche (25) des Arretierungselementes (24) als Kante (31) ausgebildet ist.

5. Einführhilfe (100) gemäß einem oder mehreren der Ansprüche 3 oder 4, **dadurch gekennzeichnet, dass** ein Winkel α zwischen einer Außenfläche (25) des Arretierungselementes (24) und der zweiten Ringfläche (30) in einem Bereich von etwa 50° bis etwa 86° liegt.

6. Einführhilfe (100) gemäß einem oder mehreren der Ansprüche 3 bis 5, **dadurch gekennzeichnet, dass** die erste Ringfläche (28) einen größeren Durchmesser aufweist als die zweite Ringfläche (30).

7. Einführhilfe (100) gemäß einem oder mehreren der vorhergehenden Ansprüche, **dadurch gekennzeichnet, dass** der Kopfbereich (20) ein Vorarretierungselement (26) umfasst, welches benachbart dem Arretierungselement (24) angeordnet ist.

8. Einführhilfe (100) gemäß einem oder mehreren der vorhergehenden Ansprüche, **dadurch gekennzeichnet, dass** das Anschlagelement (22) und/oder das Vorarretierungselement (26) zylinderförmig ausgebildet ist.

9. Einführhilfe (100) gemäß einem oder mehreren der vorhergehenden Ansprüche, **dadurch gekennzeichnet, dass** das Anschlagelement (22) und/oder das Vorarretierungselement (26) koaxial zu dem Stab (12) und der durch diesen verlaufenden Längsachse (11) ausgebildet ist.

10. Einführhilfe (100) gemäß einem oder mehreren der vorhergehenden Ansprüche, **dadurch gekennzeichnet, dass** unterhalb des Griffbereiches (20) am Stab (12) mindestens eine Materialanhäufung (32.1, 32.2) angeordnet ist.

11. Einführhilfe (100) gemäß Anspruch 10, **dadurch gekennzeichnet, dass** die mindestens eine Materialanhäufung (32.1, 32.2) unmittelbar an eine Unterfläche (29) des Anschlagelementes (22) angrenzt.

12. Einführhilfe (100) gemäß einem oder mehreren der vorhergehenden Ansprüche, **dadurch gekennzeichnet, dass** diese eine Langhülse (60), in die die stabförmige Vorrichtung (10) einführbar ist, umfasst.

13. Einführhilfe (100) gemäß Anspruch 12, **dadurch gekennzeichnet, dass** die Langhülse (60) in einem Ausführendbereich (64) elastisch ausgebildet ist.

14. Einführhilfe (100) gemäß einem oder mehreren der Ansprüche 12 und 13, **dadurch gekennzeichnet, dass** in einem Einführendbereich (62) mindestens ein Aufnahmeschlitz (66) für eine Stimmprothese (80) angeordnet ist.

15. Einführhilfe (100) gemäß einem oder mehreren der Ansprüche 12 bis 14, **dadurch gekennzeichnet, dass** eine Wandung (68) der Langhülse (60) konisch auf den Ausführendbereich (64) zulaufend ausgebildet ist.

16. Stabförmige Vorrichtung (10) für eine Stimmprothese (80) gemäß einem der Ansprüche 1 bis 11.

## Claims

1. Insertion aid (100) for a voice prosthesis (80), comprising a rod-shaped device (10) with a head region (20) located on a first end (14) of a rod (12), comprising a stop element (22) for a voice prosthesis (80) and an adjoining retention element (24) in the form of an inverted truncated cone for a voice prosthesis (80), wherein a head surface of the retention element (24), formed as a truncated cone, is arranged facing away from the stop element (22) with its larger diameter, and wherein a first annular surface (28) is formed between the stop element (22) and the retention element (24), which extends radially in relation to a longitudinal axis (11) of the rod (12).

2. Insertion aid (100) according to claim 1, **characterized in that** the first annular surface (28) is formed by the stop element (22).

3. Insertion aid (100) according to any one or more of the preceding claims, **characterized in that** the retention element (24) comprises a second annular surface (30).

4. Insertion aid (100) according to any one or more of the preceding claims, **characterized in that** a transition in the form of a rim (31) is formed between the second annular surface (30) and an outer surface (25) of the retention element (24).

5. Insertion aid (100) according to either or both of the claims 3 or 4, **characterized in that** an angle α is formed, in a range of approximately 50° to approximately 86°, between an outer surface (25) of the retention element (24) and the second annular surface (30).

6. Insertion aid (100) according to any one or more of the claims 3 to 5, **characterized in that** the first annular surface (28) has a greater diameter than the second annular surface (30).

7. Insertion aid (100) according to any one or more of the preceding claims, **characterized in that** the head region (20) comprises a pre-retention element (26) adjacent to the retention element (24).

8. Insertion aid (100) according to any one or more of the preceding claims, **characterized in that** the stop element (22) and/or the pre-retention element (26) are cylindrical.

9. Insertion aid (100) according to any one or more of the preceding claims, **characterized in that** the stop element (22) and/or the pre-retention element (26) are coaxial to the rod (12) and the longitudinal axis (11) running through it.

10. Insertion aid (100) according to any one or more of the preceding claims, **characterized in that** there is at least one material accumulation (32.1, 32.2) beneath the gripping region (20) on the rod (12).

11. Insertion aid (100) according to claim 10, **characterized in that** the at least one material accumulation (32.1, 32.2) borders directly on a lower surface (29) of the stop element (22).

12. Insertion aid (100) according to any one or more of the preceding claims, **characterized in that** it comprises a sleeve (60), into which the rod-shaped device (10) can be inserted.

13. Insertion aid (100) according to claim 12, **characterized in that** the sleeve (60) is elastic in an extraction region (64).

14. Insertion aid (100) according to either or both of the claims 12 and 13, **characterized in that** at least one receiving slit (66) for a voice prosthesis (80) is arranged in an insertion region (62).

15. Insertion aid (100) according to any one or more of the claims 12 to 14, **characterized in that** a wall (68) of the sleeve (60) tapers conically toward the extraction region (64).

16. Rod-shaped device (10) for a voice prosthesis (80) according to any of the claims 1 to 11.

## Revendications

1. Dispositif d'aide à l'insertion (100) d'une prothèse phonatoire (80), comprenant un dispositif en forme de tige (10) avec une zone de tête (20) disposée à une première extrémité (14) d'une tige (12), celle-ci comprenant un élément de butée (22) pour une prothèse phonatoire (80) et un élément de verrouillage (24) pour une prothèse phonatoire (80) se raccordant à celui-ci et formant un tronc de cône posé sur la tête, dans lequel une surface de tête de l'élément de verrouillage (24) en forme de tronc de cône est disposée à l'opposé du plus grand diamètre de l'élément de butée (22), et dans lequel, entre l'élément de butée (22) et l'élément de verrouillage (24), est formée une première surface annulaire (28) qui s'étend radialement par rapport à un axe longitudinal (11) de la tige (12).

2. Dispositif d'aide à l'insertion (100) selon la revendication 1, **caractérisé en ce que** la première surface annulaire (28) est formée par l'élément de butée (22).

3. Dispositif d'aide à l'insertion (100) selon une ou plusieurs des revendications précédentes, **caractérisé en ce que** l'élément de verrouillage (24) comprend une deuxième surface annulaire (30).

4. Dispositif d'aide à l'insertion (100) selon une ou plusieurs des revendications précédentes, **caractérisé en ce qu'**une transition entre la deuxième surface annulaire (30) et une surface extérieure (25) de l'élément de verrouillage (24) forme une arête (31).

5. Dispositif d'aide à l'insertion (100) selon une ou plusieurs des revendications 3 et 4, **caractérisé en ce qu'**un angle α entre une surface extérieure (25) de l'élément de verrouillage (24) et la deuxième surface annulaire (30) est situé sur une plage d'environ 50° à environ 86°.

6. Dispositif d'aide à l'insertion (100) selon une ou plusieurs des revendications 3 à 5, **caractérisé en ce que** la première surface annulaire (28) possède un plus grand diamètre que la deuxième surface annulaire (30).

7. Dispositif d'aide à l'insertion (100) selon une ou plusieurs des revendications précédentes, **caractérisé en ce que** la zone de tête (20) comprend un élément de préverrouillage (26) qui est adjacent à l'élément de verrouillage (24).

8. Dispositif d'aide à l'insertion (100) selon une ou plusieurs des revendications précédentes, **caractérisé en ce que** l'élément de butée (22) et/ou l'élément de préverrouillage (26) sont de forme cylindrique.

9. Dispositif d'aide à l'insertion (100) selon une ou plusieurs des revendications précédentes, **caractérisé en ce que** l'élément de butée (22) et/ou l'élément de préverrouillage (26) sont coaxiaux avec la tige (12) et l'axe longitudinal (11) qui la traverse.

10. Dispositif d'aide à l'insertion (100) selon une ou plusieurs des revendications précédentes, **caractérisé en ce que** sous la zone de prise (20) sur la tige (12) est disposée au moins une accumulation de matière (32.1, 32.2).

11. Dispositif d'aide à l'insertion (100) selon la revendication 10, **caractérisé en ce que** l'au moins une accumulation de matière (32.1, 32.2) est directement contiguë à une face inférieure (29) de l'élément de butée (22).

12. Dispositif d'aide à l'insertion (100) selon une ou plusieurs des revendications précédentes, **caractérisé en ce qu'**il comprend un fourreau allongé (60) dans lequel le dispositif en forme de tige (10) peut être inséré.

13. Dispositif d'aide à l'insertion (100) selon la revendication 12, **caractérisé en ce que** le fourreau allongé (60) est formé de manière élastique dans une zone terminale d'insertion (64).

14. Dispositif d'aide à l'insertion (100) selon une ou plusieurs des revendications 12 et 13, **caractérisé en ce qu'**au moins une fente de réception (66) destinée à une prothèse phonatoire (80) est ménagée dans une zone initiale d'insertion (62).

15. Dispositif d'aide à l'insertion (100) selon une ou plusieurs des revendications 12 à 14, **caractérisé en ce qu'**une paroi (68) du fourreau allongé (60) se termine en cône sur la zone terminale d'insertion (64).

16. Dispositif en forme de tige (10) pour une prothèse phonatoire (80) selon une des revendications 1 à 11.
